# EUROPEAN PATENT APPLICATION

(11) **EP 4 641 575 A1**
(43) Date of publication of application: **29.10.2025**
(21) Application number: 23912977.8
(22) Date of filing: 28.12.2023
(51) Int. Cl.: G16B 40/20, G16B 30/00, G06N 20/20

(54) **ARTIFICIAL INTELLIGENCE-BASED METHOD AND DEVICE FOR PREDICTING SPECIES OF MICROORGANISM**

(30) Priority: 29.12.2022 KR 20220188509
(71) Applicant: CJ Olivenetworks Co., Ltd., Seoul 04626 (KR)
(72) Inventor: SOHN, Jongsoo, Seoul 04626 (KR); KIM, Jae Kwang, Seoul 04626 (KR); JO, Seung Kyu, Seoul 04626 (KR)
(74) Representative: BCKIP Part mbB
(86) International application number: PCT/KR2023/021844
(87) International publication number: WO 2024/144303

(57) **Abstract**

The present invention relates to an artificial intelligence-based method and device for predicting the species of a microorganism. Specifically, the present invention relates to a method and device that enable more efficient prediction of the species of a microorganism by incorporating an artificial intelligence (AI) language model in a massive DNA database.

## Description

### TECHNICAL FIELD

The present invention relates to an artificial intelligence-based method and device for predicting the species of a microorganism. Specifically, the present invention relates to a method and device that enable more efficient prediction of the species of a microorganism by incorporating an artificial intelligence (AI) language model in a massive DNA database.

### BACKGROUND ART

In order to confirm the effect of distribution of intestinal microorganisms on a human body, accurate classification of the microorganisms should be preceded. Classification of microorganisms is generally accomplished through comparison of base sequences of genes, and in the case of microorganisms that can be cultured and isolated, the classification can be accomplished by comparing the base sequences with a previously known DNA database using the entire DNA base sequence or ribosomal RNA. On the other hand, in the case of microorganisms that cannot be cultured, it needs to determine the species for short read data that can be obtained through various sequencing techniques, but this is a very difficult problem.

Conventional methods of classifying species of microorganisms use a Local Alignment search tool such as basic local alignment search tool (Blast) to find hit sequences from a known DNA database, and then compare similarity between the sequences using an Average Nucleotide Identity (ANI) value. However, as the size of the DNA database increases, such a conventional method is inefficient since it needs a lot of resources and time and comparison with the DNA database every time.

### DISCLOSURE OF INVENTION

### TECHNICAL PROBLEM

An object of the present invention is to solve the problems described above by incorporating an artificial intelligence language model in a massive DNA database, and provide a method of predicting the species of a microorganism more efficiently and a device therefor.

Meanwhile, the technical problems of the present invention are not limited to the technical problems mentioned above, and unmentioned other technical problems can be clearly understood by those skilled in the art from the following descriptions.

### TECHNICAL SOLUTION

To accomplish the above object, according to one aspect of the present invention, there is provided an artificial intelligence-based method for predicting a species of a microorganism, the method comprising the steps of: performing learning of an artificial intelligence model for predicting the species of the microorganism; acquiring a whole genome sequence (WGS) of the microorganism; parsing the whole genome sequence; inputting the parsed whole genome sequence into the learned artificial intelligence model; and predicting the species of the microorganism on the basis of an output of the artificial intelligence model.

According to an embodiment, the step of performing learning of an artificial intelligence model may include the steps of: preparing data for learning of the artificial intelligence model; performing learning of a tokenizer specialized for analysis of a DNA sequence on the basis of the data; performing learning of a pre-trained language model (PLM) specialized for analysis of a DNA sequence on the basis of the tokenizer; and performing learning of a downstream task for predicting the species of the DNA sequence on the basis of the PLM.

According to an embodiment, the step of preparing data may include the steps of: acquiring whole genome sequence data for learning of the artificial intelligence model; parsing the whole genome sequence data; and preparing the parsed whole genome sequence data as data for learning of the artificial intelligence model.

According to an embodiment, the step of performing learning of a tokenizer may include the step of training the tokenizer on the basis of byte pair encoding (BPE), WordPiece, SentencePiece, and k-mer.

According to an embodiment, data used for learning of the PLM may be data excluding data used for learning of the tokenizer.

According to an embodiment, data used for learning of the downstream task may be data excluding data used for learning of the PLM.

According to an embodiment, the step of parsing the whole genome sequence of a microorganism may include the step of parsing the whole genome sequence of the microorganism in a length of 2,000 to 2,500 base pairs (bp).

According to an embodiment, the step of predicting the species of the microorganism may include the steps of: calculating a probability value for each class of the parsed whole genome sequence; calculating a probability value distribution for each class of the whole genome sequence on the basis of the probability value; and predicting a species of the microorganism having the whole genome sequence on the basis of the probability value distribution.

According to an embodiment, the step of predicting a species of the microorganism having the whole genome sequence on the basis of the probability value distribution may include the steps of: calculating an area of a section larger than or equal to a preset threshold value in the probability value distribution for each class; and predicting a species related to a class corresponding to the probability value distribution, of which the area of a section larger than or equal to a preset threshold value is the largest, as the species of the microorganism.

According to another aspect of the present invention, there is provided an artificial intelligence-based device for predicting a species of a microorganism, the device comprising a central processing unit and a memory, wherein the central processing unit executes instructions stored in the memory to perform an artificial intelligence-based method for predicting a species of a microorganism, the artificial intelligence-based method for predicting a species of a microorganism may include the steps of: performing learning of an artificial intelligence model for predicting the species of the microorganism; acquiring a whole genome sequence (WGS) of the microorganism; parsing the whole genome sequence; inputting the parsed whole genome sequence into the learned artificial intelligence model; and predicting the species of the microorganism on the basis of an output of the artificial intelligence model.

### ADVANTAGEOUS EFFECTS

According to the present invention, as an artificial intelligence-based language model specialized for analysis of a DNA sequence is incorporated, the species of a microorganism can be predicted efficiently from the information on DNA sequence of a new microorganism.

Meanwhile, the effects of the present invention are not limited to those mentioned above, and unmentioned other technical effects will be clearly understood by those skilled in the art from the following descriptions.

### BRIEF DESCRIPTION OF THE DRAWINGS

FIG. 1 is a view showing the overall configuration of an artificial intelligence (AI)-based method for predicting the species of a microorganism according to an embodiment of the present invention.
FIG. 2 is a view showing the learning process of an artificial intelligence model according to an embodiment of the present invention.
FIG. 3 is a view showing a data preparation process according to an embodiment of the present invention.
FIG. 4 is a view showing a tokenizer learning process according to an embodiment of the present invention.
FIG. 5 is a view showing a learning process of a pre-trained language model (PLM) according to an embodiment of the present invention.
FIG. 6 is a view showing a downstream task learning process according to an embodiment of the present invention.
FIG. 7 is a view schematically showing a process of predicting the species of a microorganism according to an embodiment of the present invention.
FIG. 8 is a view specifically showing a process of predicting the species of a microorganism according to an embodiment of the present invention.
FIG. 9 is a view showing the overall process of performing learning of an artificial intelligence model and predicting the species of a microorganism according to an embodiment of the present invention.

### BEST MODE FOR CARRYING OUT THE INVENTION

Details of the objects and technical configurations of the present invention and operational effects according thereto will be more clearly understood by the following detailed description based on the drawings attached in the specification of the present invention. An embodiment according to the present invention will be described in detail with reference to the accompanying drawings.

The embodiments disclosed in this specification should not be construed or used as limiting the scope of the present invention. For those skilled in the art, it is natural that the description including the embodiments of the present specification have various applications. Accordingly, any embodiments described in the detailed description of the present invention are illustrative for better describing of the present invention, and are not intended to limit the scope of the present invention to the embodiments.

The functional blocks shown in the drawings and described below are merely examples of possible implementations. Other functional blocks may be used in other implementations without departing from the spirit and scope of the detailed description. In addition, although one or more functional blocks of the present invention are expressed as separate blocks, one or more of the functional blocks of the present invention may be combinations of various hardware and software configurations that perform the same function.

In addition, the expressions including certain components are expressions of "open type" and only refer to existence of corresponding components, and should not be construed as excluding additional components.

Furthermore, when a certain component is referred to as being "connected" or "coupled" to another component, it may be directly connected or coupled to another component, but it should be understood that other components may exist in between. For reference, in this detailed description, the expressions 'performing learning of' and 'train' are used together, and it should be understood that both expressions are intended to describe the process of training an algorithm (model).

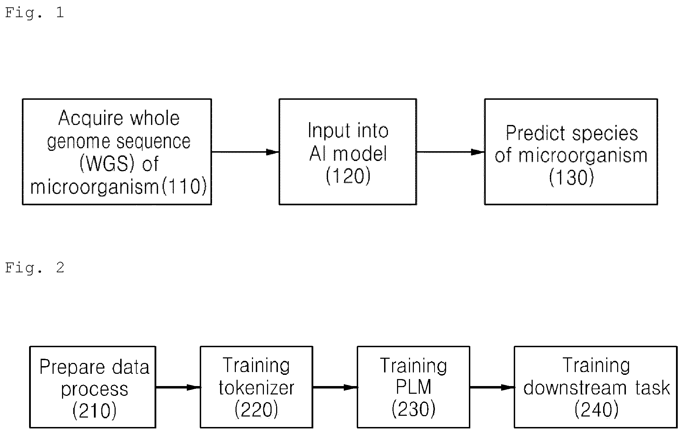

FIG. 1 is a view showing the overall configuration of an artificial intelligence (AI)-based method for predicting the species of a microorganism according to an embodiment of the present invention.

Referring to FIG. 1, an artificial intelligence-based method for predicting the species of a microorganism according to the present invention may include a process of acquiring the whole genome sequence (WGS) of a microorganism for performing species prediction thereon (110), a process of inputting the whole genome sequence of the microorganism into a learned artificial intelligence model (120), and a process of predicting the species of the microorganism on the basis of the output of the artificial intelligence model (130).

It can be understood that each step and process for performing the artificial intelligence-based method for predicting the species of a microorganism according to various embodiments of the present invention, which will be described below with reference to the drawings, is performed by an artificial intelligence-based device for predicting the species of a microorganism unless otherwise specified. In the following descriptions of the present invention, it can be understood that the "device" means an artificial intelligence-based device for predicting the species of a microorganism according to various embodiments of the present invention unless otherwise defined specifically. When the device is implemented in the form of a PC, the device may include a central processing unit and a memory. At this point, the central processing unit may also be referred to as a controller, a microcontroller, a microprocessor, a microcomputer, or the like. In addition, the central processing unit may be implemented by hardware, firmware, software, or a combination thereof. When the central processing unit is implemented using hardware, it can be implemented as an application-specific integrated circuit (ASIC), a digital signal processor (DSP), a digital signal processing device (DSPD), a programmable logic device (PLD), a field programmable gate array (FPGA), or the like. When the central processing unit is implemented using firmware or software, the firmware or software may be configured to include modules, procedures, functions, and the like that perform the functions or operations described above. In addition, the memory may be implemented as Read Only Memory (ROM), Random Access Memory (RAM), Erasable Programmable Read Only Memory (EPROM), Electrically Erasable Programmable Read-Only Memory (EEPROM), flash memory, Static RAM (SRAM), a Hard Disk Drive (HDD), a Solid-State Drive (SSD), or the like.

As shown in FIG. 1, the present invention provides a method of predicting the species of a corresponding microorganism from the DNA sequence of a newly acquired single microorganism using a learned artificial intelligence model, and a device therefor. Therefore, in order to implement the present invention, a learning process of an appropriate artificial intelligence model and a process of performing prediction of a species from the DNA sequence of a new microorganism using the learned artificial intelligence model are required.

First, the learning process of an artificial intelligence model will be described below with reference to the drawings.

FIG. 2 is a view showing the learning process of an artificial intelligence model according to an embodiment of the present invention. Referring to FIG. 2, the learning process of an artificial intelligence model may include a data preparation process 210, a tokenizer learning process 220, a pre-trained language model (PLM) learning process 230, and a downstream task learning process 240 for classifying the species of a short microorganism DNA sequence. The data preparation process 210 is a process of preparing DNA sequence information of a microorganism required for learning of the artificial intelligence model acquired from the Global Open Database or through experiments. The tokenizer learning process 220 is a learning process of a tokenizer specialized for parsing the DNA sequence obtained in the data preparation process 210. The PLM learning process 230 is a learning process of a pre-learning model specialized for DNA sequence. The downstream task learning process 240 is a process of performing fine-tuning on the learned PLM model.

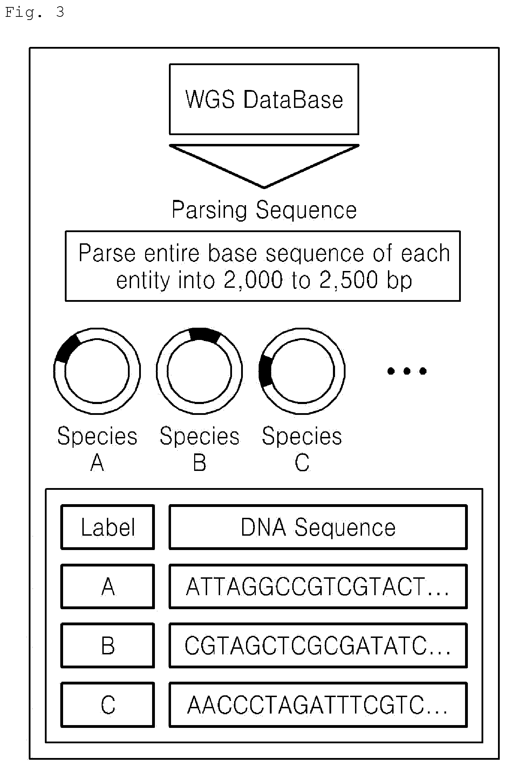

FIG. 3 is a view showing a data preparation process according to an embodiment of the present invention.

Referring to FIG. 3, in the data preparation process 210, the device may acquire DNA sequence information of a microorganism from the Global Open Database or through experiments. Here, the DNA sequence information acquired from the Global Open Database or through experiments may be a whole genome sequence (WGS). Meanwhile, the DNA sequence information is used as an input for learning of an artificial intelligence model. However, since the length of the DNA sequence of a microorganism is very long ranging from about 1,000,000 to 15,000,000 and is not constant, it may not be appropriate to use the entire length as an input of an artificial intelligence model. Therefore, a process of parsing the acquired DNA sequence in short length is necessary. The present invention may perform parsing so that the DNA sequence information of a microorganism acquired from the Global Open Database or through experiments is segmented into a plurality of sequences having a short length. For example, the DNA sequence information of a microorganism acquired from the Global Open Database or through experiments may be parsed in a length of 2,000 to 2,500 base pairs (bp). Here, the bp represents a unit of the number of base pairs, and 1 bp means one base pair. At this point, label processing may be performed together to indicate from which species each sequence segmented through the parsing is segmented. For example, as shown in FIG. 3, parsing may be performed on the acquired whole genome sequence (WGS) to include a DNA sequence "ATTAGGCCGTCGTACT..." labeled as "A", a DNA sequence "CGTAGCTCGCGATATC..." labeled as "B", and a DNA sequence "AACCCTAGATTTCGTC..." labeled as "C". The device may use the parsed DNA sequence as data for learning of the artificial intelligence model thereafter. After the data preparation process 210, the tokenizer learning process 220 may be performed based on the sequences segmented to have a short length through the parsing.

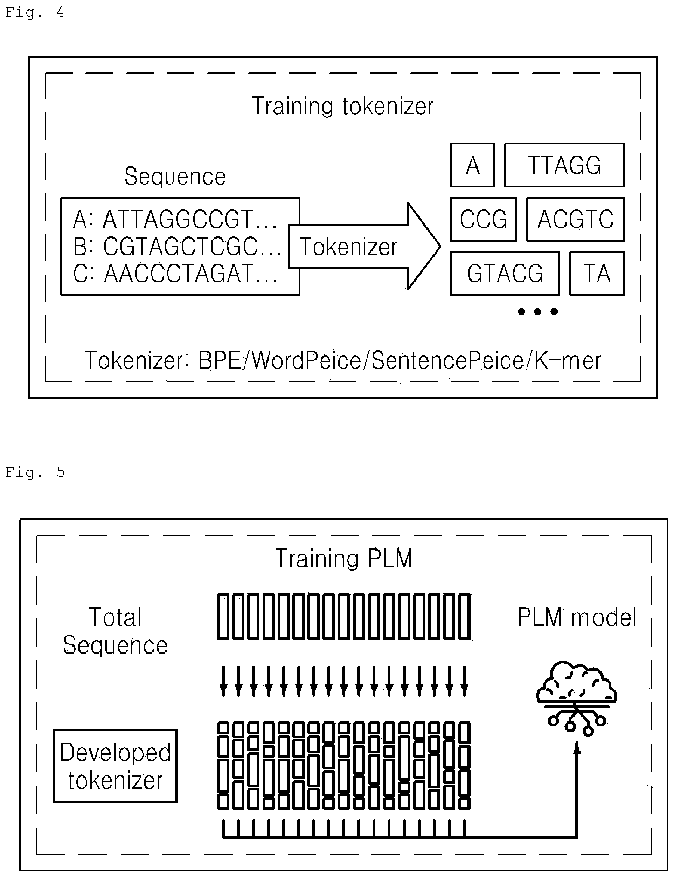

FIG. 4 is a view showing a tokenizer learning process according to an embodiment of the present invention.

Although tokenizers used in the existing language models are learned based on the language system (spacing, words, prefixes, suffixes, etc.) used by humans, since DNA sequences have a system different from the language system used by humans, a new language model and tokenizer specialized for analysis of a DNA sequence are required. In the present invention, a tokenizer may be learned by applying a k-mer method together with byte pair encoding (BPE), WordPiece, and SentencePiece used in the existing language models. Here, the k-mer means all possible subsequences having a length of k for a given DNA sequence. For example, in the data preparation process 210, 3-mers for the DNA sequence "ATTAGGCCGTCGTACT..." labeled as "A" may be ATT, TTA, TAG, AGG, GGC, GCC, CCG, CGT, GTC, TCG, CGT, GTA, TAC, ACT, ... as all possible subsequences having a length of 3, and 5-mers may be ATTAG, TTAGG, TAGGC, AGGCC, GGCCG, GCCGT, CCGTC, CGTCG, GTCGT, TCGTA, CGTAC, GTACT, ... as all possible subsequences having a length of 5. Such a k-mer analysis is a method used in genome sequence analysis, and in the present invention, a tokenizer specialized for parsing DNA sequence may be learned using k-mers together with byte pair encoding (BPE), WordPiece, and SentencePiece. Through the tokenizer learning process 220, a tokenizer specialized for analysis of a DNA sequence can be acquired, and the pre-trained language model (PLM) learning process 230 may be performed as a language model specialized for analysis of a DNA sequence on the basis of the learned tokenizer.

FIG. 5 is a view showing a learning process of a pre-trained language model (PLM) according to an embodiment of the present invention.

Referring to FIG. 5, for learning of a PLM specialized for DNA sequence, a language model capable of masking a part of the DNA sequence on the basis of a tokenizer learned through the tokenizer learning process 220 and predicting the masked part well may be trained (220). Here, the better a PLM can predict a masked part well, the higher the performance of the PLM. A Bidirectional Encoder Representations from Transformer (BERT), a Robust BERT (RoBERT), or the like may be used as a language model used for PLM learning. At this point, data used for training the tokenizer in the tokenizer learning process 220 may be excluded from the learning data used for PLM learning. Through the PLM learning process 230 described above, a language model specialized for analysis of a DNA sequence may be learned, and the downstream task learning process 240 of updating parameters needs to be performed using the learned language model so that the parameters may be used for prediction of the species of a microorganism, which is an object of the present invention.

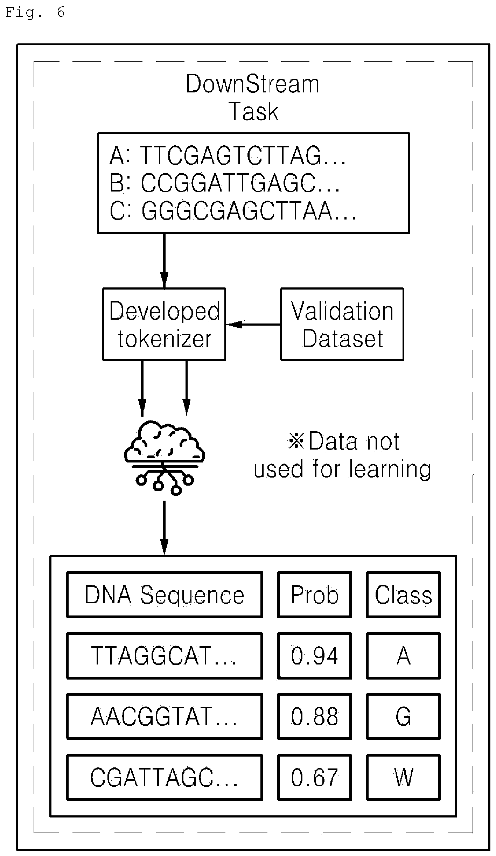

FIG. 6 is a view showing a downstream task learning process according to an embodiment of the present invention.

The downstream task to be performed in the present invention is predicting a species corresponding to a DNA sequence having a short length from the DNA sequence, and in the downstream task learning process 240, fine-tuning may be performed to predict the species of a microorganism using the PLM learned through the PLM learning process 230. The DNA sequences segmented in short length and the labeling-processed data may be used as learning data for fine-tuning, and at this point, learning data used in the PLM learning process 230 may be excluded. When learning is completed, whether the species of a DNA sequence having a short length is well predicted can be verified based on a validation dataset prepared in advance. Here, the validation dataset prepared in advance may be different data from the learning data used in the downstream task learning process 240.

When the downstream task learning process 240 is completed, the learning process of an artificial intelligence model according to an embodiment of the present invention is completed. The artificial intelligence model learned according to the method described above may be used for classification of species of a DNA sequence having a short length, and thus may be used as a prediction model for distribution of microorganisms of short DNA reads data.

A learning process of an artificial intelligence model is described above, and a process of predicting the species of a new microorganism using the learned artificial intelligence model is described below with reference to the drawings.

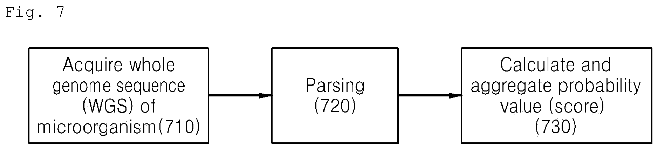

FIG. 7 is a view schematically showing a process of predicting the species of a microorganism according to an embodiment of the present invention.

As described above, the learned artificial intelligence model may be used for classification of the species of a DNA sequence having a short length, and the whole genome sequence (WGS) of a microorganism has a very large size ranging from about 1,000,000 to 15,000,000. Therefore, in order to predict the species of a new microorganism using the WGS of the microorganism, a process of comprehensively determining a result of predicting species for DNA sequences having a short length predicted using the learned artificial intelligence model is needed. Therefore, the process of predicting the species of a microorganism according to an embodiment of the present invention may include a process of acquiring the whole genome sequence (WGS) of a new microorganism (710), a process of parsing the acquired WGS (720), and a process of calculating and aggregating probability values (scores) for the parsed sequences using the learned artificial intelligence model (730), as shown in FIG. 7.

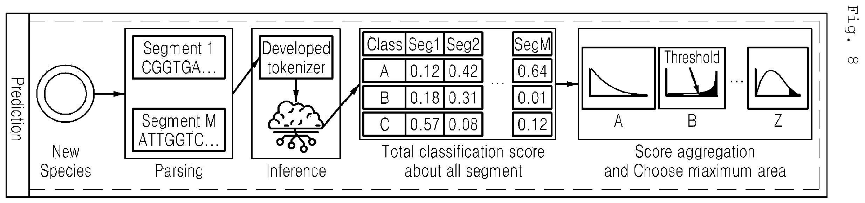

FIG. 8 is a view specifically showing a process of predicting the species of a microorganism according to an embodiment of the present invention.

Referring to FIG. 8, in order to predict the species of a new microorganism, a process similar to that performed in the data preparation process 210 included in the learning process of an artificial intelligence model may be performed. That is, the DNA sequence of a new microorganism to be predicted may be parsed in short length. For example, the DNA sequence of a new microorganism is acquired in the form of a whole genome sequence (WGS) having a length of about 1,000,000 to 15,000,000, and the DNA sequence may be parsed into short sequences having a length of 2,000 to 2,500 bp. For example, in the example of FIG. 8, the DNA sequence of a new microorganism may be parsed into short sequences including Seg1 (CGGTGA...), ..., SegM (ATTGGTC...). The parsed short sequences are input into the artificial intelligence model of which the learning has been completed according to the method described above through FIGS. 2 to 7, and probability values (scores) for all classes of the parsed short sequences may be calculated through the artificial intelligence model. Here, different classes may correspond to species of different microorganisms, respectively. In the case the example of FIG. 8, probability values for classes A, B, and C of Seg1 may be calculated as 0.12, 0.18, and 0.57, respectively, probability values for classes A, B, and C of Seg2 may be calculated as 0.42, 0.31, and 0.08, respectively, and probability values for classes A, B, and C of SegM may be calculated as 0.64, 0.01, and 0.12, respectively. The corresponding probability values (scores) are calculated as many as the number of parsed short sequences for each class in this way, and the probability value distribution (score distribution) of the entire DNA sequence (i.e., WGS) may be obtained based on the corresponding probability values. In order to determine a final class by comparing the probability value distribution (score distribution) for each species, an area value of a section larger than a specific threshold is calculated after the threshold is determined, and a species corresponding to a class with the largest calculated area value may be determined as a species predicted finally. In the case of the example of FIG. 8, a class with the largest area of a section larger than the threshold value corresponds to "B", and therefore, the species corresponding to class "B" may be finally predicted as the species of the new input microorganism.

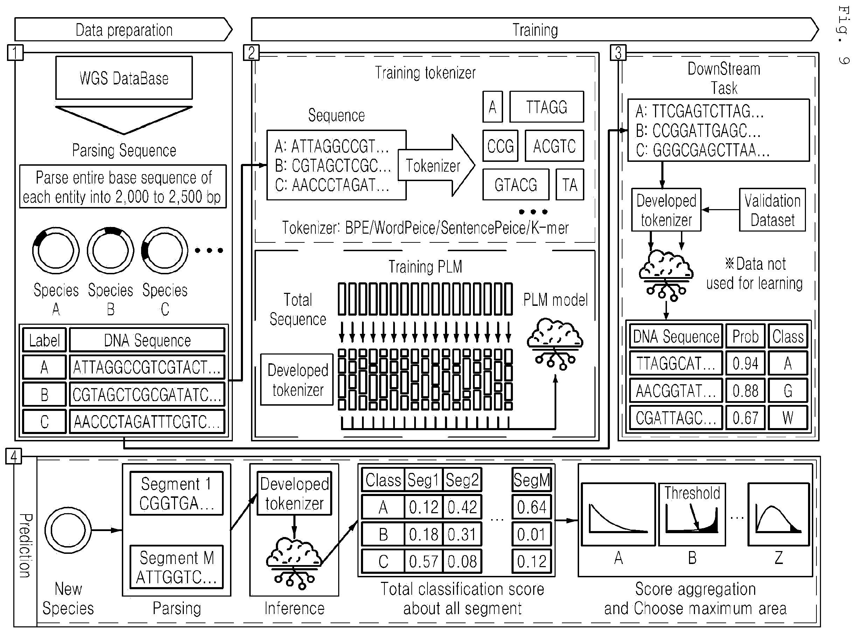

FIG. 9 is a view showing the overall process of performing learning of an artificial intelligence model and predicting the species of a microorganism according to an embodiment of the present invention.

Referring to FIG. 9, before the process of predicting the species of a microorganism, a process for data preparation and learning of an artificial intelligence model may be performed first. As described with reference to FIG. 3, the device may acquire DNA sequence information in the form of a whole genome sequence (WGS) through the Global Open Database or experiments, and prepare data for learning of an artificial intelligence model by parsing the acquired DNA sequence information to have a short length. Thereafter, the device may train an artificial intelligence model used for predicting the species of a new microorganism through tokenizer learning, PLM learning, and downstream task learning. Since preparation of data and learning of the artificial intelligence model have been described above in detail through FIGS. 2 to 6, detailed description thereof will be omitted herein. When learning of the artificial intelligence model is completed on the basis of the prepared data, the species of a new microorganism may be predicted using the learned artificial intelligence model. When the DNA sequence of a new microorganism, of which the species is to be predicted, is acquired in the form of a whole genome sequence (WGS), the acquired DNA sequence may be parsed to have a short length. Probability values (scores) of the sequences parsed to have a short length are calculated for all classes through the artificial intelligence model, and a probability value distribution (score distribution) for all classes of the entire DNA sequence is calculated based on the probability values before parsing is performed, and the species of the microorganism can be finally predicted using the probability value distribution for all classes. Since prediction of the species of a microorganism has also been described above in detail through FIGS. 7 and 8, detailed description thereof will be omitted herein.

In the specific embodiments of the present disclosure described above, the components included in the disclosure are expressed in a singular or plural form according to the presented specific embodiment. However, the singular or plural expressions are selected to be suitable for a presented situation for the convenience of explanation, and the present disclosure is not limited to singular or plural components, and even a component expressed in a plural form may be configured in a singular form, or even a component expressed in a singular form may be configured in a plural form.

Meanwhile, although specific embodiments are described in the detailed description of the present disclosure, it is obvious that various modifications are possible without departing from the scope of the present disclosure. Therefore, the scope of the present disclosure should not be limited to the described embodiments, and should be determined by the equivalents of the scope of the claims, as well as the scope of the claims described below.

## Claims

1. An artificial intelligence-based method for predicting a species of a microorganism, the method comprising the steps of:
performing learning of an artificial intelligence model for predicting the species of the microorganism;
acquiring a whole genome sequence (WGS) of the microorganism;
parsing the whole genome sequence;
inputting the parsed whole genome sequence into the learned artificial intelligence model; and
predicting the species of the microorganism on the basis of an output of the artificial intelligence model.

2. The method according to claim 1, wherein the step of performing learning of an artificial intelligence model includes the steps of:
preparing data for learning of the artificial intelligence model;
performing learning of a tokenizer specialized for analysis of a DNA sequence on the basis of the data;
performing learning of a pre-trained language model (PLM) specialized for analysis of a DNA sequence on the basis of the tokenizer; and
performing learning of a downstream task for predicting the species of the DNA sequence on the basis of the PLM.

3. The method according to claim 2, wherein the step of preparing data includes the steps of:
acquiring whole genome sequence data for learning of the artificial intelligence model;
parsing the whole genome sequence data; and
preparing the parsed whole genome sequence data as data for learning of the artificial intelligence model.

4. The method according to claim 2, wherein the step of performing learning of a tokenizer includes the step of training the tokenizer on the basis of byte pair encoding (BPE), WordPiece, SentencePiece, and k-mer.

5. The method according to claim 2, wherein data used for learning of the PLM is data excluding data used for learning of the tokenizer.

6. The method according to claim 2, wherein data used for learning of the downstream task is data excluding data used for learning of the PLM.

7. The method according to claim 1, wherein the step of parsing the whole genome sequence of a microorganism includes the step of parsing the whole genome sequence of the microorganism in a length of 2,000 to 2,500 base pairs (bp).

8. The method according to claim 1, wherein the step of predicting the species of the microorganism includes the steps of:
calculating a probability value for each class of the parsed whole genome sequence;
calculating a probability value distribution for each class of the whole genome sequence on the basis of the probability value; and
predicting a species of the microorganism having the whole genome sequence on the basis of the probability value distribution.

9. The method according to claim 8, wherein the step of predicting a species of the microorganism having the whole genome sequence on the basis of the probability value distribution includes the steps of:
calculating an area of a section larger than or equal to a preset threshold value in the probability value distribution for each class; and
predicting a species related to a class corresponding to the probability value distribution, of which the area of a section larger than or equal to a preset threshold value is the largest, as the species of the microorganism.

10. An artificial intelligence-based device for predicting a species of a microorganism, the device comprising a central processing unit and a memory, wherein
the central processing unit executes instructions stored in the memory to perform an artificial intelligence-based method for predicting a species of a microorganism, the artificial intelligence-based method for predicting a species of a microorganism includes the steps of:
performing learning of an artificial intelligence model for predicting the species of the microorganism;
acquiring a whole genome sequence (WGS) of the microorganism;
parsing the whole genome sequence;
inputting the parsed whole genome sequence into the learned artificial intelligence model; and
predicting the species of the microorganism on the basis of an output of the artificial intelligence model.

11. The method according to claim 10, wherein the step of performing learning of an artificial intelligence model includes the steps of:
preparing data for learning of the artificial intelligence model;
performing learning of a tokenizer specialized for analysis of a DNA sequence on the basis of the data;
performing learning of a pre-trained language model (PLM) specialized for analysis of a DNA sequence on the basis of the tokenizer; and
performing learning of a downstream task for predicting the species of the DNA sequence on the basis of the PLM.

12. The device according to claim 11, wherein the step of preparing data includes the steps of:
acquiring whole genome sequence data for learning of the artificial intelligence model;
parsing the whole genome sequence data; and
preparing the parsed whole genome sequence data as data for learning of the artificial intelligence model.

13. The device according to claim 11, wherein the step of performing learning of a tokenizer includes the step of training the tokenizer on the basis of byte pair encoding (BPE), WordPiece, SentencePiece, and k-mer.

14. The device according to claim 11, wherein data used for learning of the PLM is data excluding data used for learning of the tokenizer.

15. The device according to claim 11, wherein data used for learning of the downstream task is data excluding data used for learning of the PLM.

16. The device according to claim 10, wherein the step of parsing the whole genome sequence of a microorganism includes the step of parsing the whole genome sequence of the microorganism in a length of 2,000 to 2,500 base pairs (bp).

17. The device according to claim 10, wherein the step of predicting the species of the microorganism includes the steps of:
calculating a probability value for each class of the parsed whole genome sequence;
calculating a probability value distribution for each class of the whole genome sequence on the basis of the probability value; and
predicting a species of the microorganism having the whole genome sequence on the basis of the probability value distribution.

18. The device according to claim 17, wherein the step of predicting a species of the microorganism having the whole genome sequence on the basis of the probability value distribution includes the steps of:
calculating an area of a section larger than or equal to a preset threshold value in the probability value distribution for each class; and
predicting a species related to a class corresponding to the probability value distribution, of which the area of a section larger than or equal to a preset threshold value is the largest, as the species of the microorganism.
